(19) Europäisches Patentamt
European Patent Office
Office européen des brevets

(11) **EP 2 706 976 B1**

(12) **FASCICULE DE BREVET EUROPEEN**

(45) Date de publication et mention
de la délivrance du brevet:
**02.03.2016 Bulletin 2016/09**

(21) Numéro de dépôt: **12719767.1**

(22) Date de dépôt: **11.05.2012**

(51) Int Cl.:
*A61K 8/64* $^{(2006.01)}$ *A61K 8/73* $^{(2006.01)}$
*A61Q 19/00* $^{(2006.01)}$ *A61P 17/00* $^{(2006.01)}$
*A61K 38/16* $^{(2006.01)}$ *A61K 36/48* $^{(2006.01)}$
*A61Q 17/04* $^{(2006.01)}$ *A61Q 19/08* $^{(2006.01)}$
*A61Q 19/10* $^{(2006.01)}$

(86) Numéro de dépôt international:
**PCT/EP2012/058798**

(87) Numéro de publication internationale:
**WO 2012/156319 (22.11.2012 Gazette 2012/47)**

(54) **NOUVEL ACTIF ANTI-ROUGEURS ET COMPOSITIONS LE COMPRENANT**

NEUER WIRKSTOFF GEGEN RÖTUNGEN UND DIESE ENTHALTENDE ZUSAMMENSETZUNG

NOVEL ANTI-REDNESS ACTIVE AGENT AND COMPOSITIONS COMPRISING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.05.2011 FR 1154181**

(43) Date de publication de la demande:
**19.03.2014 Bulletin 2014/12**

(73) Titulaire: **Laboratoires Expanscience**
**92400 Courbevoie (FR)**

(72) Inventeurs:
• **MSIKA, Philippe**
**F-78000 Versailles (FR)**
• **BAUDOUIN, Caroline**
**F-78120 Rambouillet (FR)**
• **MENU, Franck**
**F-28260 Sorel Moussel (FR)**

(74) Mandataire: **Regimbeau**
**139, rue Vendôme**
**69477 Lyon Cedex 06 (FR)**

(56) Documents cités:
WO-A1-00/62789      WO-A1-2010/052327
FR-A1- 2 855 050    FR-A1- 2 885 301

**Description**

La présente invention est relative à la prévention et au traitement des rougeurs de la peau, souvent considérées comme inesthétiques. Elle concerne l'emploi de l'arabinogalactane et de compositions le comprenant, pour la prévention et le traitement des rougeurs. Une composition particulièrement efficace contre les rougeurs de la peau comprend de l'arabinogalactane et des peptides de lupin. Une méthode de prévention et/ou traitement des rougeurs de la peau est présentée, comprenant l'administration à une personne sujette aux rougeurs cutanées d'une composition comprenant de l'arabinogalactane.

**INTRODUCTION**

Les rougeurs de la peau sont des troubles cutanés, essentiellement présentes sur le visage, qui peuvent avoir des origines diverses. Elles peuvent être de courte durée (flushs) ou permanentes. Des critères secondaires peuvent se manifester comme des sensations de brûlures, de picotements, des plaques rouges, une sécheresse cutanée, un oedème facial. Selon la sévérité de ces rougeurs, le handicap esthétique peut être très intense et s'accompagner de conséquences sociales désastreuses. On estime que 10% de la population Européenne est concernée.

Les personnes présentant des rougeurs cutanées présentent généralement une sensibilité anormale de la peau à l'environnement, et à de nombreux facteurs comme : les boissons chaudes, l'alcool, les changements de température, les aliments épicés, l'exercice, le stress et les fortes émotions, le soleil...

L'apparition de ces rougeurs est mal connue et multifactorielle : elle implique des facteurs génétique, inflammatoire et vasculaire.

Récemment, l'équipe du Pr Gallo a découvert [1] et mis en évidence l'implication d'une réponse immune innée altérée dans l'initiation des troubles cutanés, et propose différents mécanismes moléculaires et acteurs clés interconnectés dans l'apparition des rougeurs : 1) immunité innée altérée ; 2) troubles vasculaires ; 3) microbes ; 4) stress oxydatif et soleil.

**1- Dérégulation de la réponse immune innée**

Le système immun inné implique un système de reconnaissance incluant les récepteurs TLRs (Toll-Like Receptors), capables de répondre à de nombreux stimuli environnementaux comme les UV, les microbes, les agressions physiques ou chimiques. Suite à l'étape de reconnaissance, la réponse immune innée se met en place avec une production contrôlée de médiateurs tels que des cytokines et des peptides antimicrobiens (PAMs). Parmi les PAMs, les cathélicidines LL37 possèdent des propriétés antimicrobiennes, mais sont aussi impliquées dans la cicatrisation et dans l'initiation de la réponse immune acquise par des propriétés de chimiotactisme. Pour libérer la forme active de cathélicidine LL37, la pro-forme doit être clivée par une enzyme de type sérine protéase appelée kallikréine 5 ou encore SCTE (stratum corneum tryptic enzyme).

Dans des biopsies de sujets présentant des rougeurs (notamment dues à une dilatation des vaisseaux sanguins superficiels à la surface de la peau), la quantité de cathélicidines LL37 est fortement augmentée. En comparaison à la peau normale, les cathélicidines LL37 ne sont pas seulement présentes en quantité plus abondante mais ont un profil peptidique différent, en conséquence ces cathélicidines sont appelés « variants LL37 ». Ces variants LL37 présentent des propriétés vasoactives et pro-inflammatoires en induisant la production d'IL8 et de certains médiateurs pro-angiogéniques [2,3]. La présence de ces variants LL37 en quantité importante dans les peaux sujettes aux rougeurs s'explique par une production anormale de la protéase kallikréine 5 qui mature les pro-formes en formes actives de LL37 [4]. Ces protéases kallikréines, sont également capables de dégrader les cornéodesmosomes et sont donc impliquées dans la desquamation. Elles sont capables également de digérer des éléments de la matrice extracellulaire (MEC) comme les collagènes I, II, III, IV, la fibronectine et la laminine. Ainsi, considérant que la production de KLK5 est augmentée dans certains troubles cutanés, ces protéases pourraient jouer un rôle important dans les réactions inflammatoires associées et affecter la matrice dermique et vasculaire.

Tout ceci a été confirmé expérimentalement par l'injection des peptides LL37 ou de l'enzyme KLK5 dans la peau de souris induisant une inflammation cutanée (érythème, vasodilatation) mimant les changements observés dans l'erythro-couperose [5].

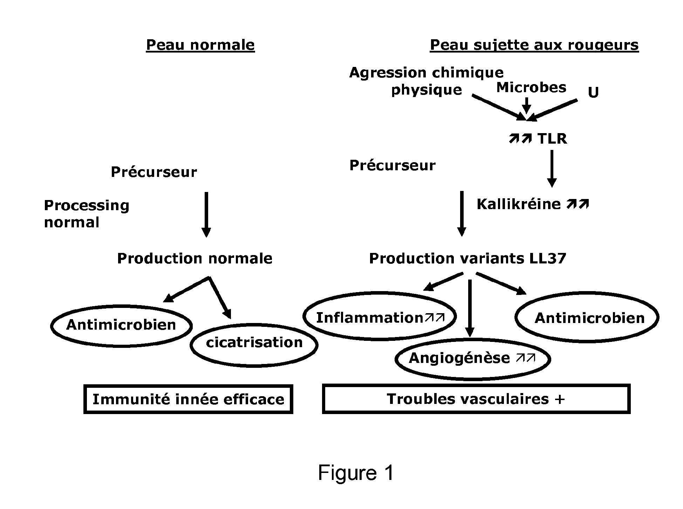

La même équipe vient de montrer que l'expression du récepteur TLR2 est anormalement augmentée [6], ceci augmentant la susceptibilité à certains stimuli et menant à une production augmentée de kallikréines et de cathélicidines et donc à une réponse exacerbée que les mêmes stimuli n'auraient pas induits chez des sujets normaux. La figure 1 illustre l'implication de la réponse immune innée.

## 2- Troubles vasculaires

De nombreuses personnes présentent par épisode des « flushes » ; ceci mène à l'hypothèse d'une hyper-réactivité vasculaire qui jouerait un rôle important dans ce phénomène inesthétique. Quelques études montrent une augmentation du flux sanguin dans les lésions cutanées de sujets présentant des rougeurs cutanées [8]. Les facteurs qui déclenchent ces flushes comme le stress émotionnel, la nourriture épicée, les boissons chaudes, les températures environnementales élevées et la ménopause, aggravent l'apparition des rougeurs et soutiennent la théorie vasculaire [8].

L'erythro-couperose désigne la présence de rougeurs à la surface de la peau associées à des petits vaisseaux capillaires principalement localisés au niveau du visage ou parfois du décolleté. Il existe indéniablement une néoangiogenèse (néo vascularisation ou synthèse de nouveaux vaisseaux sanguins) dans l'apparition de ces rougeurs, aboutissant à des télangiectasies (petits vaisseaux apparents) [9]. Le stimulus initial reste mal connu, bien que le soleil ou d'autres facteurs environnementaux soient des candidats suggérés pour causer ce phénomène.

Sous l'effet de réactions vasomotrices répétées, se produisent des lésions du réseau lymphatique responsables d'une inflammation persistante, gênante pour la personne.

Ceci induit l'activation de cellules capables de produire des molécules pro-inflammatoires, des facteurs de croissance angiogéniques comme le VEGF ou FGF2 et des protéases comme les métalloprotéinases ou MMPs. Les MMPs dégradent la matrice extracellulaire du tissu cutané et libèrent ainsi des facteurs de croissance angiogéniques, et dégradent les parois vasculaires permettant aux cellules endothéliales de migrer [10]. La néoangiogenèse consiste en la formation de nouveaux vaisseaux à partir de vaisseaux existants *via* des modifications morphologiques et fonctionnelles des cellules endothéliales vasculaires. Un niveau d'expression élevé de VEGF a été mesuré dans la peau de personnes présentant des rougeurs [11, 12].

Par ailleurs, les cathélicidines pourraient être aussi déclencheurs de l'hypervascularité et de l'angiogénèse dans l'apparition des rougeurs cutanées [5, 13].

Les étapes clés de l'angiogénèse sont les suivantes :

→ Libération par les cellules de l'environnement de médiateurs délétères : cytokines, protéases comme MMPs, et facteurs de croissance pro-angiogéniques ;
→ L'équilibre bascule en faveur des médiateurs pro-angiogéniques : VEGF / FGF2 (ou bFGF) qui sont :

- Sécrétés par les cellules de l'environnement,
- Libérés de la Matrice Extracellulaire par les MMPs ;

→ Activation des cellules endothéliales
→ Dégradation de la paroi vasculaire par les MMPs
→ Migration et prolifération des cellules endothéliales
→ Formation nouveau vaisseau

Dans l'apparition des rougeurs cutanées, l'exposition solaire, le stress oxydatif et certains microbes sont des facteurs déclenchant et exacerbant le phénomène disgracieux.

## 3- Rôle des bactéries et parasites

Le *demodex folliculorum* est un parasite saprophyte (acarien) colonisant le follicule pilosébacé et dont la densité peut-être augmentée lors de rougeurs cutanées. Une bactérie *Bacillus oleronius* a été isolée de Demodex : 1 parasite contient 40 bactéries. Cette bactérie *B. oleronius* induit la prolifération des cellules mononucléaires et provoque une réponse inflammatoire chez les personnes présentant des rougeurs associées à des pustules. De plus, *B. oleronius* libère des molécules qui stimulent l'immunité innée *via* les TLRs.

## 4- Stress oxydatif et soleil

Les radicaux libres oxygénés (ROS) sont augmentés dans les lésions d'erythro-couperose, mais la localisation précise des ces ROS est indéterminée. La source cellulaire des ROS serait les leucocytes et les kératinocytes. Suite à la libération des ROS, une cascade de signaux cellulaires se met en place pour induire la production de médiateurs pro-inflammatoires (TNFα par les kératinocytes) et de MMPs (par les fibroblastes). Ainsi, les ROS contribuent à l'endommagement du matériel dermique et vasculaire et à la réponse inflammatoire.

Les UV causent les flushes et aggravent les rougeurs cutanées. Les UV induisent l'angiogénèse chez la souris et stimulent la production de VEGF, de FGF2 dans les kératinocytes et la peau de souris. Les UV génèrent également des ROS et stimulent la production de médiateurs inflammatoires (IL8) et de MMPs. Les UV stimulent la voie TLR2 et notamment induisent la production de LL37. Les UV altèrent qualitativement et quantitativement le tissu conjonctif dermique qui est le support du réseau vasculaire. Les médiateurs (inflammatoires, angiogéniques et MMPs) produits par les UV détruisent les parois vasculaires, stimulent les cellules endothéliales permettant une extravasation, une vasodilatation et une inflammation. Ainsi les anomalies de matériel élastique (élastose) et collagénique constitue un mauvais support aux vaisseaux et contribue à la formation d'érythème et de télangiectasies. Les UV induisent donc des lésions dermiques et vasculaires [1].

## ART ANTERIEUR

Peu de composés sont connus pour la prévention et/ou le traitement des rougeurs de la peau. L'huile de chanvre est connue pour atténuer les symptômes. En cas d'attaque bactérienne, des crèmes antibiotiques peuvent être prescrites. Des stéroïdes topiques peuvent avoir un effet à court terme, mais à long terme ils ont une tendance à aggraver les symptômes de la couperose. Un traitement au laser permet de détruire les vaisseaux superficiels de l'épiderme du patient pendant quelques années : ce traitement onéreux doit cependant être répété régulièrement.

Des compositions contenant du chitosan et un amide d'acide dicarboxylique à chaîne courte/moyenne, formant un film après application sur la peau, ont été décrites pour prévenir et traiter les rougeurs cutanées (WO 2009/150257). Des compositions à base de modulateurs des récepteurs de PACAP (Pituitary adenylate cyclase activating polypeptide) ont également été proposées pour diminuer les rougeurs cutanées (WO 2010/007175). Des compositions à base de plantes ont également été décrites, telles que :

- Compositions comprenant des extraits de thé vert et/ou de soja, en association avec des extraits de feuilles de ginkgo bilboa (FR2885301) ;
- Compositions comprenant de la caféine (FR2855050) ;
- Compositions comprenant des peptides de lupin qui possèdent des propriétés d'inhibition des métalloprotéases (WO 00/62789).

### Les Arabinogalactanes

Les arabinogalactanes (aussi appelé galactoarabinanes) sont des polysaccharides. Elles sont présentes en quantités variables dans de multiples végétaux, champignons et bactéries. Les arabinogalactanes sont des fibres solubles naturelles qui peuvent être extraites à partir de bactéries ou de plantes telles que le café ou les mélèzes.

L'arabinogalactane est un polymère composé de deux types de saccharides, le galactose et l'arabinose, dans un ratio de 6 :1 respectivement.

Des méthodes d'extraction de l'arabinogalactane ont été décrites, en particulier à partir de café (EP 1 600 461, WO 2007/099997) et de mélèze (EP 0 866 808). Dans le commerce, les arabinogalactanes les plus courantes sont celles issues de mélèze, un arbre particulièrement riche en arabinogalactanes.

L'arabinogalactane a de nombreux effets sur le métabolisme des mammifères ; à titre d'exemple, nous pouvons citer les effets suivants :

- Les arabinogalactanes sont des fibres non digestibles ayant une action pré biotique, c'est-à-dire qui favorisent la prolifération, dans le tube digestif, de bactéries utiles à l'organisme.
- L'arabinogalactane a la réputation de stimuler le système immunitaire. La demande EP 1 600 461 revendique en particulier l'ajout d'arabinogalactane dans des aliments afin d'obtenir des « aliments santé ». L'arabinogalactane stimule la sécrétion d'interleukine-12 ce qui favorise la réparation de l'ADN (FR2836378).
- La demande WO 2010/020379 décrit des compositions comprenant de l'arabinogalactane pour la prévention et le traitement des maladies allergiques et inflammatoires : l'arabinogalactane a la propriété de diminuer significativement la sécrétion d'IgE.
- La demande WO 2010/052327 décrit l'arabinogalactane comme étant un composé actif pour la prévention des vergetures. En effet, l'arabinogalactane présente les propriétés suivantes :

  - il favorise le renouvellement cellulaire, notamment des fibroblastes ;
  - il stimule l'expression du collagène de types I et III ;
  - il inhibe la sécrétion d'IL-1$\beta$.

De manière tout à fait surprenante, les inventeurs de la présente demande ont mis en évidence un autre effet bénéfique de l'arabinogalactane sur le traitement et la prévention des rougeurs de la peau. L'arabinogalactane présente en effet des propriétés intéressantes sur la limitation de la prolifération des cellules endothéliales lors des épisodes de rougeurs cutanées. Son action anti-rougeurs est par ailleurs stimulée lorsqu'il est utilisé en combinaison avec des peptides de lupin.

## DESCRIPTION DE L'INVENTION

La présente invention est relative à une méthode de prévention et/ou de traitement des rougeurs de la peau, comprenant l'administration d'une composition comprenant de l'arabinogalactane, à une personne susceptible de former ou ayant des rougeurs.

La composition peut comprendre en outre d'autres composés anti-rougeurs, tels que notamment des peptides de lupin. Une telle association présente des effets synergiques sur les différents phénomènes moléculaires intervenant dans l'apparition des rougeurs.

## DESCRIPTION DETAILLEE DE L'INVENTION

De par ses propriétés régulatrices des phénomènes d'immunité innée et de néoangiogenèse, l'arabinogalactane permet de diminuer la réactivité de certains types de peaux susceptibles de développer des rougeurs, et ainsi de prévenir et traiter l'apparition de ces rougeurs. L'arabinogalactane étant issue de plante, un des avantages de l'invention est de proposer aux personnes sujettes aux rougeurs cutanées, et donc ayant une peau particulièrement sensible, une composition comprenant essentiellement des produits naturels.

Les 'rougeurs de la peau' désignent des érythèmes faciaux et des télangiectasies, de toutes origines.

Par l'expression « prévention des rougeurs de la peau», on entend selon la présente invention une action permettant d'éviter ou à tout le moins de réduire la formation de rougeurs inesthétiques, par application de la composition, avant et au cours d'un évènement connu comme pouvant provoquer l'apparition de rougeurs, tel qu'une exposition solaire ou un stress.

Par l'expression « traitement des rougeurs de la peau » on entend selon la présente invention une action permettant de diminuer les symptômes inesthétiques par application de la composition sur les rougeurs apparues.

Par l'expression « composition comprenant de l'arabinogalactane », on entend selon la présente invention que le composé 'arabinogalactane' est le principe actif de la composition, c'est-à-dire qu'il a une action propre, en tant que tel, sur la prévention et le traitement des rougeurs de la peau. Comme cela est démontré dans les exemples, l'arabinogalactane agit sur différents facteurs moléculaires impliqués dans l'apparition des rougeurs : inhibition de l'expression de LL37, inhibition de la prolifération des cellules endothéliales et inhibition de l'expression du VEGF.

Par «personne susceptible de former ou ayant des rougeurs », on entend une personne jugeant inesthétiques et handicapantes des rougeurs sur le visage, quel que soit le stade auquel ces rougeurs peuvent être classifiées d'un point de vue clinique.

Selon un aspect préféré de l'invention, ladite composition est administrée par voie topique, en particulier sur des zones de la peau susceptibles de former des rougeurs et notamment le visage.

La composition peut également être administrée par voie orale, notamment sous forme de comprimés, gélules, capsules molles, dragées, émulsions, gels, ou sous forme de compléments ou produits alimentaires.

Selon un aspect préféré de l'invention, l'arabinogalactane utilisée est extraite de mélèze. Un procédé d'extraction est décrit en particulier dans la demande de brevet EP 0 866 808. Selon un aspect préféré de l'invention, le mélèze dont est issu l'arabinogalactane est cultivé de manière raisonnée et durable. L'arabinogalactane peut également être extraite d'herbes, notamment de vulpin des prés, de fléole des prés, de dactyle ou de seigle.

Préférentiellement, la composition anti-rougeurs selon l'invention comprend de l'arabinogalactane dans une proportion comprise entre 0,01% et 10% en poids, avantageusement entre 1 et 5%, et plus préférentiellement dans une proportion d'environ 2% en poids par rapport au poids total de la composition.

Selon un aspect préféré de l'invention, la composition anti-rougeurs comprend au moins un autre agent anti-rougeurs, choisi en particulier parmi la liste suivante: les peptides de lupin, le perméthol, la génistéine, l'esculoside, le sulfate de dextran, l'hespéridine méthylchalcone, les rétinoïdes, la licochalcone, l'oxyméthazoline, la kinétine, l'extrait de réglisse, la vitamine P like, l'extrait de petit houx, le *Sophora japonica,* l'extrait d'Hamamélis, le ruscus, les antibiotiques tels que la doxycycline, les polyphénols dont les tanins, les acides phénoliques, les anthocyanes, les procyanidols, les flavonoïdes avec par exemple la quercétine, les extraits de thé vert, de fruits rouges, de cacao, de raisin, de *Passiflora incarnata,* de *Citrus.*

Chacun de ces composés à une action propre visant à amplifier ou favoriser l'action de l'arabinogalactane sur les rougeurs de la peau.

Selon un aspect préféré de l'invention, ladite composition comprend de l'arabinogalactane et des peptides de lupin.

Par « peptides de lupin », on entend toute préparation comprenant un extrait de lupin enrichi en peptides, telles que par exemple les préparations suivantes :

- les hydrolysats protéiques de lupin Structurine® et Anagéline®, commercialisés par la société Silab ;
- l'hydrolysat peptidique de lupin 'Sweet Blue Lupine Peptides®', commercialisé par la société Oat Cosmetics.

Une préparation préférée de peptides de lupin est la préparation commercialisée par la société Laboratoires Expanscience, obtenue selon le procédé décrit dans la demande WO2005/102259.

En particulier, la composition selon l'invention comprend entre 0,01% et 10% d'arabinogalactane, et entre 0,01% et 10% de peptides de lupin.

Une composition préférée comprend 2% d'arabinogalactane et 0,2% de peptides de lupin.

Selon un aspect préféré de l'invention, la composition anti-rougeurs comprend en outre au moins un agent apaisant et restructurant, choisi en particulier parmi les agents suivants:

- un extrait peptidique de quinoa
- une oxazoline
- un concentrat d'huile de tournesol
- un acétate de vitamine E
- ainsi que les divers mélanges de ces composés.

Chacun de ces composés à une action propre visant à amplifier, compléter ou favoriser l'action de l'arabinogalactane et optionnellement des peptides de lupin sur les peaux sujettes à rougeurs.

Par « extrait peptidique de quinoa » on entend notamment un extrait à partir de graines de quinoa tel que décrit dans la demande internationale WO2008/080974, cet extrait étant caractérisé en ce qu'il comprend 10 à 90% en poids de peptides et de 10 à 50% en poids de sucres par rapport au poids total dudit extrait et étant utilisé pour ses propriétés réparatrices de la barrière et anti-inflammatoires.

Les concentrats d'huile de tournesol pouvant être utilisés dans le cadre de la présente invention en association avec l'arabinogalactane sont avantageusement des concentrats de tournesol linoléiques, tels que l'actif commercialisé par les Laboratoires Expanscience, Soline® (cf. la demande internationale WO 01/21150) notamment pour leur activité restructurante et anti-irritante et/ou apaisante.

Les oxazolines pouvant être utilisées dans le cadre de la présente invention en association avec l'arabinogalactane sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide® (cf. demandes internationales WO2004050052, WO2004050079, et WO2004112741). Elles sont particulièrement utiles pour leur activité anti-inflammatoire et/ou anti-irritante et/ou apaisante.

La composition selon l'invention peut en outre comprendre au moins un composé choisi dans le groupe constitué par les agents hydratant et/ou cicatrisant et/ou restruturant et/ou anti-irritant et/ou apaisant et/ou anti-oxydant et/ou anti-vieillissement .

Les actifs hydratants / émollients peuvent être la glycérine ou ses dérivés, l'urée, l'acide pyrrolidone carboxylique et ses dérivés, l'acide hyaluronique de tout poids moléculaire, les glycosaminoglycanes et tout autres polysaccharides d'origine marine, végétale ou biotechnologique comme par exemple la gomme xanthane, le fucogel®, des acides gras comme l'acide laurique, l'acide myristique, les acides gras poly- et mono-insaturés de type oméga 3, 6 et 7, 9 comme l'acide linoléique et acide palmitoléique, certains beurre comme le beurre de karité.

Les agents cicatrisants et/ou restructurants de la barrière cutanée pouvant être utilisés en association sont avantageusement le panthénol (vitamine B5), l'oxyde de zinc, l'acide madécassique ou asiatique, le sulfate de dextran, le coenzyme Q10, la glucosamine et ses dérivés, la chondroïtine sulfate et globalement les glucosaminoglycanes, le sulfate de dextran, les céramides, le cholestérol, le squalane, les phospholipides. Peuvent également être utilisés des agonistes PPARs (rosiglitazone, pioglitazone), RXR, LXR.

Les agents anti-inflammatoires et/ou anti-irritants et/ou apaisants peuvent être l'acide glycyrrhétinique (les dérivés de réglisse) avec ses sels et esters, l'acide lipoïque, le beta-carotène, la vitamine B3 (niacinamide, nicotinamide), la vitamine E, la vitamine C, la vitamine B12, le lycopène ou la lutéine, les eaux de source ou thermales (eau d'Avène, eau de la Roche Posay, eau de Saint Gervais, eau d'Uriage, eau de Gamarde), ou encore la disulone topique, ou les médicaments anti-inflammatoires stéroïdiens (AIS), tels que les corticoïdes, ou non-stéroïdiens (AINS). On peut citer également les isoflavones, notamment de soja, comme par exemple la génistéine/génistine, daidzéine/daidzine,

Les antioxydants pouvant être utilisés en association sont avantageusement choisis dans le groupe constitué par les thiols et les phénols, par les dérivés de réglisse comme l'acide glycyrrhétinique avec ses sels et esters, l'alpha bisabolol, l'acide lipoïque, la vitamine B12, la vitamine B3 (niacinamide, nicotinamide), les vitamines C, les vitamines E, le coenzyme Q10, le krill, le glutathion, le BHT pour butylhydroxytoluène, le BHA pour butylhydroxyanisol, le lycopène ou la lutéine, le beta-carotène. Dans le groupe des antioxydants, on trouve aussi les substances anti-glycation telles que la carnosine ou certains peptides, la n-acétyl-cystéine, ainsi que les enzymes antioxydants ou antiradicalaires comme la SOD (super oxyde dismutase), la catalase, la glutathion peroxydase, la thioredoxine reductase et leurs agonistes.

Les agents anti-âge peuvent être la vitamine C, l'acide hyaluronique de tout poids moléculaire, les rétinoïdes comme le rétinol, le rétinal et les rétinoïdes ; en particulier les rétinoïdes non aromatiques tels le rétinaldéhyde, la trétinoïne, l'isotrétinoïne et l'acide rétinoïque 9-cis, la vitamine A, les rétinoïdes monoaromatiques tels l'étrétinate, l'all-trans acitretine et le motrerinide, et les rétinoïdes polyaromatiques tels que l'adapalène, le tazarotène, le tamibarotène et l'arotinoïde methyl sulfone.

De plus, la composition selon l'invention peut comprendre également des filtres et écrans solaires minéraux ou organiques (pigmentaires ou ultrafins), des composés antifongiques, des conservateurs et des agents anti-bactériens.

Les actifs protecteurs solaires pouvant être utilisés en association sont avantageusement des filtres ou écrans solaires UVB et/ou UVA ; tels les écrans ou filtres minéraux et/ou organiques connus de l'homme du métier qui adaptera leur choix et leurs concentrations en fonction du degré de protection recherché. A titre d'exemple d'actifs protecteur solaire, on peut notamment citer le dioxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S), l'octocrylène, le butyl méthoxydibenzoylméthane, l'acide terephthalylidene dicamphor sulfonique, le 4-méthylbenzylidène de camphre, le benzophénone, l'éthylhexyl méthoxycinnamate, l'éthylhexyl diméthyl PABA, le diéthylhexyl butamido triazone.

Les composés antifongiques pouvant être utilisés en association sont avantageusement l'econazole et le ketoconazole.

Les conservateurs pouvant être utilisés en association sont par exemple ceux généralement utilisés en cosmétique ou en nutraceutique, les molécules à activité anti-bactérienne (pseudo-conservateurs) tels que les dérivés capryliques comme par exemple le capryloyl glycine et le glycéryl caprylate, tels que l'hexanediol, et le sodium levulinate, les dérivés de zinc et de cuivre (gluconate et PCA), la phytosphingosine et ses dérivés, le peroxyde de benzoyle, la piroctone olamine, le pyrithione zinc, le sulfure de sélénium. Les conservateurs antiseptiques pouvant être utilisés en association sont par exemple le triclosan, la chlorhéxidine, les ammoniums quaternaires.

Les antibiotiques pouvant être utilisés en association sont avantageusement l'acide fucidique, la pénicilline, les tétracyclines, la pristinamycine, l'érythromycine, la clindamycine, la mupirocine, la minocycline, la doxycycline. Les agents anti-viraux pouvant être utilisés en association sont avantageusement l'acyclovir et le valacyclovir.

Parmi les actifs recommandés en association avec l'extrait selon l'invention, on peut citer les extraits végétaux, en particulier :

- Les huiles végétales telles que les huiles de soja et/ou l'huile de colza, l'huile d'avocat (WO2004/012496, WO2004/012752, WO2004/016106, WO2007/057439), l'huile de lupin, avantageusement l'huile de lupin blanc doux (WO 98/47479), l'huile de pépin de courge (sébo-régulateur), l'huile de melaleuca (anti-pelliculaire), l'huile de bourrache (anti-pelliculaire), l'huile de cade ;
- l'oléodistillat ou les concentrats d'huile végétale ou animale, notamment de tournesol, plus avantageusement des concentrats de tournesol linoléiques, tels que l'actif commercialisé par les Laboratoires Expanscience, Soline® (cf. la demande internationale WO 01/21150), d'avocat, de colza, de maïs ou de palme, utiles notamment pour leur activité hydratante et/ou émolliente, cicatrisante et/ou restructurante de la barrière cutanée, anti-inflammatoire et/ou anti-irritante et/ou apaisante ;
- les insaponifiables de végétaux ou d'huile végétale, avantageusement l'Avocadofurane® (furanes d'avocat, pouvant être obtenus par le procédé décrit dans la demande internationale WO 01/21605), les insaponifiables d'avocat et de soja, plus particulièrement un mélange d'insaponifiables d'avocat furaniques et d'insaponifiables de soja, dans un rapport respectif d'environ 1/3-2/3 (tels que Piasclédine®), les insaponifiables de soja (tel qu'obtenu selon le procédé décrit dans la demande internationale WO01/51596), les insaponifiables stéroliques (insaponifiables dont la teneur en stérols, en méthylstérols et en alcools triterpèniques est comprise entre 20 et 95 % en poids, de préférence 45-65 % en poids, par rapport au poids total de l'insaponifiable), les phytostérols, les esters de stérols et les dérivés vitaminiques, utiles notamment pour leur activité cicatrisante et/ou restructurante de la barrière cutanée, anti-âge, antiinflammatoire
- les peptides ou complexes d'acides aminés végétaux, en particulier les peptides d'avocat (tel que ceux décrits dans la demande internationale WO2005/105123), les peptides de lupin (tels que ceux obtenus selon le procédé décrit dans la demande WO2005/102259), les peptides de quinoa (tel que ceux décrits dans la demande internationale WO2008/080974), les peptides de maca tel que ceux décrits dans la demande internationale WO2004/112742), les peptides de soja fermenté ou non, les peptides de riz (tel que ceux décrits dans la demande internationale WO 2008/009709), utiles notamment pour leur activité hydratante et/ou émolliente (avocat), kératorégulatrice (lupin, quinoa), cicatrisante et/ou restructurante de la barrière cutanée (maca, quinoa, soja), anti-inflammatoire et/ou anti-irritante et/ou apaisante (lupin, quinoa, schizandra), antioxydante (avocat), anti-âge (lupin, maca, avocat), pigmentant (riz) ;

- les sucres de végétaux, en particulier les sucres d'avocat (tel que ceux décrits dans la demande WO2005/115421), utiles notamment pour leur action kératorégulatrice, cicatrisante et/ou restructurante de la barrière cutanée, anti-inflammatoire et/ou anti-irritante et/ou apaisante ;
- le 5 alpha Avocuta® (butyl avocadate), inhibiteur de la 5-alpha réductase (voir WO 01/52837 et WO 02/06205) et régulateur de la sécrétion séborrhée se trouvant augmentée dans l'acné ou les pellicules ;
- un extrait de fruit d'avocat enrichi en polyphénols (tel que décrit dans la demande FR 1 061 055) ;
- un extrait de feuilles de maca (tel que décrit dans la demande FR 1 061 047) ;
- un extrait de *gynandropsis gynandra* (tel que décrit dans la demande FR 1 061 051);
- le lupéol (FR 2 822 821, FR 2 857 596) utile notamment pour favoriser la cicatrisation,
- un extrait total de lupin (tel que ceux décrits dans la demande internationale WO2005/102259), particulièrement adapté pour le traitement des irritations ;
- les isoflavones comme par exemple les isoflavones de soja ;
- un beurre de cupuaçu, particulièrement apprécié pour ses propriétés hydratantes ;
- un extrait peptidique et osidique de graines *d'Acacia macrostachya* (FR 0958525), adapté pour ses propriétés hydratantes
- un extrait de graines de *Vigna unguiculata* (FR 0958529)
- un extrait de fruit de *Schizandra sphenanthera* (tel que ceux décrits dans les demandes FR 0955344, WO 2011/012612) et un extrait peptidique de schizandra (FR 0955343 et WO 2011/012615) adapté pour ses propriétés anti-inflammatoires et anti-acnéiques.

La composition topique selon l'invention comprend également un véhicule approprié qui peut être tout véhicule parmi ceux connus de l'homme du métier en vue d'obtenir une composition cosmétique utilisable selon l'invention, en particulier sous forme d'une crème, d'une lotion, d'un gel, d'un spray, d'un patch, d'une eau, d'une pommade, de lait ou d'huile, éventuellement sous la forme d'une émulsion, avec en outre des composants connus de l'homme du métier pour améliorer, modifier ou stabiliser la composition d'un point de vue cosmétique.

Pour l'ingestion par voie orale, de nombreuses formes de réalisation et notamment de compléments alimentaires sont possibles. Leur formulation est réalisée par les procédés usuels pour produire des comprimés, des gélules, des capsules molles, des dragées, des émulsions, des gels. En particulier, l'arabinogalactane et les autres actifs de l'invention peuvent être incorporés dans toutes formes de compléments alimentaires ou d'aliments enrichis, par exemple, des barres alimentaires, des poudres compactées ou non, des boissons, des produits laitiers et en particulier des yaourts et des yaourts à boire. Les poudres peuvent être diluées dans de l'eau, des sodas, des jus de fruits, des produits laitiers ou à base de soja, de riz, ou être incorporées dans des barres alimentaires.

Les conditions opératoires pour préparer ces compositions selon l'invention font partie des connaissances générales de l'homme du métier.

La présente invention est également relative à une utilisation d'arabinogalactane pour prévenir et/ou traiter l'apparition des rougeurs sur la peau.

La présente invention est également relative à une utilisation d'une combinaison d'arabinogalactane et de peptides de lupin pour prévenir et/ou traiter l'apparition des rougeurs sur la peau.

La présente invention est également relative à une composition thérapeutique comprenant de l'arabinogalactane pour son utilisation dans la prévention et/ou le traitement des rougeurs cutanées.

L'invention est également relative à une méthode de prévention et/ou de traitement des rougeurs cutanées, comprenant l'administration par voie topique ou orale d'une composition comprenant de l'arabinogalactane.

La présente invention est également relative à une composition comprenant de l'arabinogalactane et des peptides de lupin pour son utilisation dans la prévention et/ou le traitement des rougeurs cutanées.

L'invention est également relative à une méthode de prévention et/ou de traitement des rougeurs cutanées, comprenant l'administration par voie topique ou orale d'une composition comprenant de l'arabinogalactane et des peptides de lupin.

La présente invention est également relative à une composition comprenant de l'arabinogalactane pour son utilisation dans la prévention et/ou le traitement de la rosacée.

En effet, les rougeurs peuvent être associées aux stades précoces de la rosacée, une affection bénigne chronique de la peau, affectant essentiellement le visage et se traduisant par des flushes, un érythème permanent, des papules, des pustules et des télangectasies. Quatre stades de la rosacée ont été proposés :

• Stade I : rosacée érythématotelangectasique : flush, érythème persistant et telangectasies ;
• Stade II : rosacée ou rosacée papulopustuleuse: érythème persistant, papules et pustules ;
• Stade III : phymas ou rosacée phymateuse ;

- Stade IV : rosacée oculaire.

L'invention est également relative à une méthode de prévention et/ou de traitement de la rosacée, comprenant l'administration par voie topique ou orale d'une composition comprenant de l'arabinogalactane.

La présente invention est également relative à une composition comprenant de l'arabinogalactane et des peptides de lupin pour son utilisation dans la prévention et/ou le traitement de la rosacée.

L'invention est également relative à une méthode de prévention et/ou de traitement de la rosacée, comprenant l'administration par voie topique ou orale d'une composition comprenant de l'arabinogalactane et des peptides de lupin.

## FIGURES

**Figure 1 :** Rôle clé de l'immunité innée dans l'apparition des rougeurs chez les personnes sujettes aux rougeurs, par rapport à la peau normale ; Revu d'après Bevins CL and Liu FT [7]

**Figure 2 :** Synergie des effets de la combinaison arabinogalactane et peptides de lupin sur l'expression de LL37 et VEGF.

## EXEMPLES

Dans les exemples in vitro, les actifs sont dilués à raison de 0,2% pour l'arabinogalactane, et 0,02% pour les peptides de lupin ; dans les compositions cosmétiques finales, les concentrations utilisées seront de dix fois supérieures (2% d'arabinogalactane, 0,2% de lupin).

## 1. Effets de l'arabinogalactane et de la combinaison arabinogalactane et peptides de lupin sur l'expression génique de la kallikréine 5 (KLK5) et de la cathélicidine (LL37)

Afin d'évaluer l'activité potentielle de l'arabinogalactane, éventuellement en association avec des peptides de lupin, dans la phase inflammatoire des rougeurs cutanées, nous avons étudié l'effet de ce complexe d'actifs sur l'expression génique de la cathélicidine LL37 et de la kallikréine 5 (KLK5) induites par un analogue de la vitamine D (calcitriol) dans des kératinocytes.

### Matériel et méthodes

Des kératinocytes épidermiques humains normaux (NHEK) ont été cultivés en milieu de culture différenciant supplémenté en $Ca^{++}$.

Les kératinocytes ont été traités pendant 24 heures avec les actifs arabinogalactane (ARG) / peptides de lupin (PLU) aux concentrations de 0,2% / 0,02% seuls ou en association ; en présence ou non de calcitriol (analogue de la vitamine D).

A la fin du traitement, les surnageants de culture ont été éliminés et les ARN totaux ont été extraits analysés et dosés. Les cDNA néo-synthétisés relatifs aux gènes d'intérêt (LL37 et KLK5) ou aux gènes de référence ont été amplifiés sélectivement par PCR en temps réel sur iQ5 [Biorad] par la technologie SybrGreen.

Pour chaque échantillon, le niveau d'expression du gène d'intérêt a été normalisé par le niveau d'expression du gène de référence le plus stable (gène GAPDH). A partir des calculs de $\Delta Ct$ ($Ct_{\text{gène intérêt}}$ - $Ct_{\text{gène de référence}}$) et de $\Delta\Delta Ct$ ($\Delta Ct_{\text{contrôl}}$ - $\Delta Ct_{\text{traité}}$), la quantité relative du gène d'intérêt a été mesurée : $QR = (1+E)^{\Delta\Delta Ct}$, en considérant que E (l'efficacité) est égale à 1, on a donc $QR = 2^{\Delta\Delta Ct}$.

La significativité des résultats a été vérifiée sur les valeurs de $\Delta Ct$, par une analyse de variance à un facteur suivie d'un test de Tuckey.

Le % inhibition de l'expression LL37 et KLK5 est ainsi calculé :

$$[(QR \text{ (stim calcitriol)} – QR \text{ (traitement actif))} / QR \text{ (stim calcitriol)}] *100$$

**Résultats**

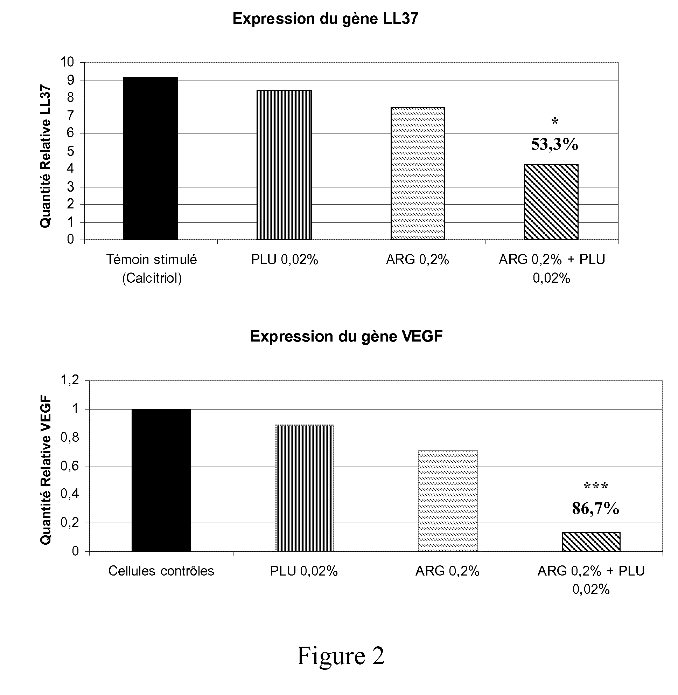

**Expression génique de LL37 (Tableau 1 et Figure 2):**

Le calcitriol a fortement et significativement stimulé l'expression génique de la LL37 par les kératinocytes (+812%, p<0,001).

L'arabinogalactane utilisé à 0,2% inhibe significativement de 18% l'expression de la LL37.

L'association d'actifs arabinogalactane + peptides de lupin inhibe de 53% l'expression génique de la LL37 induite par le calcitriol. Cette inhibition résulte de l'effet synergique de l'association de l'arabinogalactane avec les peptides de lupin. En effet, testés seuls, ces actifs inhibent plus modérément l'expression de la LL37.

**Tableau 1**

| | Moyenne $\Delta$Ct ($Ct_{LL37}$ - $Ct_{GAPDH}$) | Ecart type | $\Delta\Delta$Ct | Expression de LL37 Quantité relative | % inhibition |
|---|---|---|---|---|---|
| Cellules contrôles | 14,21 | 0,69 | | 1,00 | |
| Témoin stimulé (Calcitriol) | 11,02 | 0,28 | 3,19 | 9,12 $$$ | |
| ARG 0,2% | 11,31 | 0,57 | 2,9 | 7,46* | 18,2% |
| PLU 0,02% | 11,14 | 0,66 | 3,07 | 8,39 ns | 7,9% |
| ARG 0,2% +PLU 0,02% | 12,12 | 1,10 | 2,09 | 4,25** | 53,3% |

$$$ p<0,001 *vs* cellules contrôles
**p<0,01 ;* p<0,05 *vs* témoin stimulé ; ns : non significatif

**Expression génique de KLK5 (Tableau 2):**

Le calcitriol a fortement et significativement stimulé l'expression génique de la kallikréine 5 par les kératinocytes (+149%, p<0,001).

L'association d'actifs arabinogalactane + peptides de lupin a nettement inhibé l'expression génique de la KLK5 induite par le calcitriol (39% d'inhibition).

**Tableau 2**

| | Moyenne $\Delta$Ct ($Ct_{KLK5}$ - $Ct_{GAPDH}$) | Ecart type | $\Delta\Delta$Ct | Expression de KLK5 Quantité relative | % Inhibition |
|---|---|---|---|---|---|
| Cellules contrôles | 3,78 | 0,99 | 0 | 1,00 | |
| Témoin stimulé (Calcitriol) | 2,46 | 1,35 | 1,32 | 2,49 $$$ | |
| ARG 0,2% +PLU 0,02% | 3,18 | 1,25 | 0,6 | 1,52 | 39,3% |

$$$ p<0,001 *vs* cellules contrôles

**Conclusion**

Dans ces conditions expérimentales, l'association d'actifs arabinogalactane + peptides de lupin a permis de moduler de façon synergique l'expression de la cathélicidine LL37, ainsi que de l'enzyme responsable de sa maturation, la kallikréine 5.

Ainsi, l'association arabinogalactane / peptides de lupin contribue à moduler l'induction de la réponse immune et inflammatoire dépendante de la voie TLR2/KLK5/LL37 dans l'apparition des rougeurs cutanées.

**2. Activité sur les paramètres vasculaires : prolifération de cellules endothéliales**

**Matériel et méthodes**

Des cellules endothéliales micro-vasculaires dermiques humaines normales ont été incubées pendant 24 ou 48 heures en absence (contrôle) ou en présence des actifs arabinogalactane (ARG) / peptides de lupin (PLU) aux concentrations de 0,2% / 0,02% seuls ou en association et en présence du VEGF (Vascular Endothelial Growth Factor), activateur de référence.

A la fin de l'incubation, la viabilité des cellules a été évaluée par une méthode spectrophotométrique : Le p-nitrophényl phosphate (PNPP) est transformé en p-nitrophénol par les phosphatases des cellules viables et l'absorbance à 405 nm du p-nitrophénol est directement proportionnelle au nombre de cellules viables.

Les résultats sont exprimés sous forme de pourcentage du signal « PNPP » du contrôle sans VEGF à T0 (moyenne $\pm$ déviation standard).

La siginificativité statistique des différences entre les conditions « cell stimulées VEGF » et « VEGF + actifs » a été évaluée par une analyse de variance à un facteur suivie d'un test de Holm-Sidak.

Le % d'inhibition de la prolifération est calculé selon l'équation suivante :

$$[ \text{(traitement actif)} - \text{(stimulé VEGF)} / \text{(contrôle sans VEGF)} - \text{(stim VEGF)}] *100$$

**Résultats**

**2.1. Prolifération des cellules endothéliales en présence de VEGF (Tableau 3):**

Le VEGF, facteur angiogénique, stimule la croissance des cellules endothéliales par rapport aux cellules contrôles (non traitées au VEGF) : +78,9% (p<0,01) après 24 heures d'incubation et +159% (p<0,01) après 48 heures d'incubation.

En présence d'arabinogalactane, cette stimulation de croissance est diminuée de 17% à 24h, de preque 10% à 48h.

En présence de la combinaison arabinogalactane + peptides de lupin, la prolifération des cellules endothéliales induite par le VEGF est significativement inhibée (50% et 25% d'inhibition à 24 et 48h respectivement), avec une action synergique des deux actifs.

**Tableau 3**

| | T24h - % / contrôle (sans VEGF) T0 | % Inhibition 24h | T48h - % / contrôle (sans VEGF) T0 | % Inhibition 48h |
|---|---|---|---|---|
| Cellules contrôle sans VEGF | 163,3 $\pm$ 8 | | 162,8 $\pm$ 8,3 | |
| Témoins stimulés VEGF | 213,2 $\pm$ 10,8 | | 262,6 $\pm$ 5 | |
| ARG 0,2% | 204, 7 $\pm$ 7,1 | 17,03% | 252,9 $\pm$ 6 | 9,71% |
| PLU 0,02% | 209,1 $\pm$ 6,4 | 8,22% | 259,3 $\pm$ 7,2 | 3,31% |
| ARG 0,2% + PLU 0,02% | 187,8 $\pm$ 4,1 | 50,90% p<0,05 | 237,2 $\pm$ 9,9 | 25,45% p<0,05 |

L'arbinogalactane, en limitant la prolifération des cellules endothéliales, contribue à limiter la phase précoce de l'angiogénèse.

L'association arabinogalactane + peptides de lupin est plus efficace que l'arabinogalactane seule pour limiter cette prolifération cellulaire.

**2.2. Expression génique de marqueurs de l'angiogénèse : VEGF, FGF2, HIF1$\alpha$, thrombospondine**

Afin de comprendre l'effet anti-angiogénique de l'arabinogalactane, l'expression génique par des kératinocytes de certains marqueurs impliqués dans l'angiogénèse a été étudiée.

- L'expression de VEGF et FGF2, facteurs de croissance pro-angiogéniques : VEGF (Vascular endothelial growth factor) est le principal facteur de croissance de l'endothélium vasculaire. C'est une cytokine vasoactive et inflammatoire puissante qui agit directement sur les cellules endothéliales en stimulant leur migration et leur prolifération et favorise donc la néoangiogenèse. De plus, le VEGF entraine une augmentation de la perméabilité des cellules endothéliales dont les protéines plasmatiques vont se déverser dans la matrice extracellulaire (hyperperméabilité vasculaire). Le VEGF a des propriétés chimiotactiques permettant le recrutement des cellules inflammatoires. Le VEGF est sécrété par les kératinocytes, fibroblastes, mastocytes, cellules inflammatoires et cellules endothéliales. Le VEGF ainsi que ses récepteurs sont augmentés dans les lésions de sujets présentant des rougeurs cutanées [12].

FGF2 ou bFGF : facteur de croissance synthétisé par les kératinocytes, fibroblastes, cellules inflammatoires et cellules endothéliales et puissant régulateur de l'angiogenèse en synergie avec le VEGF.

- HIF1$\alpha$, facteur de transcription du VEGF,
- La thrombospondine, facteur anti-angiogénique.

**Matériel et méthodes**

Des kératinocytes épidermiques humains normaux (NHEK) ont été incubés en présence du complexe d'actifs arabinogalactane (ARG) / peptides de lupin (PLU) aux concentrations de 0,2% / 0,02% seuls ou en association.

A la fin du traitement, les surnageants de culture ont été éliminés et les ARN totaux ont été extraits analysés et dosés. Les cDNA néo-synthétisés relatifs aux gènes d'intérêt (VEGF, FGF2, HIF1$\alpha$, THBS1) ou aux gènes de référence ont été amplifiés sélectivement par PCR en temps réel sur iQ5 [Biorad] par la technologie SybrGreen.

Pour chaque échantillon, le niveau d'expression du gène d'intérêt a été normalisé par le niveau d'expression du gène de référence le plus stable (gène GAPDH). A partir des calculs de $\Delta Ct$ ($Ct_{\text{gène intérêt}}$ - $Ct_{\text{gène de référence}}$) et de $\Delta\Delta Ct$ ($\Delta Ct_{\text{contrôle}}$ - $\Delta Ct_{\text{traité}}$), la quantité relative du gène d'intérêt a été mesurée : QR = $(1+E)^{\Delta\Delta Ct}$, en considérant que E (l'efficacité) est égale à 1, on a donc $Q_R = 2^{\Delta\Delta Ct}$.

La significativité des résultats a été vérifiée sur les valeurs de $\Delta Ct$, par une analyse de variance à un facteur suivie d'un test de Dunnett.

Le % inhibition est calculé pour VEGF, FGF2 et HIF 1$\alpha$ :

$$[(QR\ (contrôle) - QR\ (traitement\ actif)) / QR\ (contrôle)] * 100$$

Le % stimulation est calculé pour THBS 1 :

$$[(QR\ (traitement\ actif) - QR\ (contrôle)) / QR\ (contrôle)] * 100$$

**Résultats**

**2.2.1. Expression génique du VEGF (Tableau 4 et Figure 2) :**

L'arabinogalactane inhibe significativement l'expression du VEGF de 29%.

L'association d'actifs arabinogalactane / peptides de lupin a très fortement et significativement inhibé l'expression du VEGF par des kératinocytes (86% d'inhibition) et de façon très supérieure aux actifs testés seuls. Cette inhibition résulte de l'effet synergique de l'association de l'arabinogalactane avec les peptides de lupin.

**Tableau 4**

| | Moyenne $\Delta CT$ ($CT_{VEGF}$ - $CT_{GAPDH}$) | Ecart type | $\Delta\Delta CT$ | Expression du VEGF Quantité relative | % Inhibition |
|---|---|---|---|---|---|
| Cellules contrôles | 8,53 | 0,58 | | 1 | |
| ARG 0,2% | 9,03 | 0,62 | -0,5 | 0,707** | 29,3% |
| PLU 0,02% | 8,7 | 0,98 | -0,17 | 0,889 ns | 11,1% |

(suite)

| | Moyenne $\Delta$CT (CT$_{VEGF}$ - CT$_{GAPDH}$) | Ecart type | $\Delta\Delta$CT | Expression du VEGF Quantité relative | % Inhibition |
|---|---|---|---|---|---|
| ARG 0,2% + PLU 0,02% | 11,44 | 1,34 | -2,91 | 0,133 *** | 86,7% |

*** p<0,001 ; ** p<0,01 *vs* cellules contrôles ; ns : non significatif

### 2.2.2. Expression génique du FGF2 (Tableau 5) :

L'association d'actifs arabinogalactane / peptides de lupin a totalement et significativement inhibé l'expression du FGF2 par des kératinocytes.

**Tableau 5**

| | Moyenne $\Delta$CT (CT$_{FGF2}$ - CT$_{GAPDH}$) | Ecart type | $\Delta\Delta$CT | Expression du FGF2 Quantité relative | % Inhibition |
|---|---|---|---|---|---|
| Cellules contrôles | 6,97 | 0,83 | | 1 | 1 |
| ARG 0,2% + PLU 0,02% | 14,34 | 1,37 | -7,37 | 0,006 *** | 99,4% |

*** p<0,001 *vs* cellules contrôles

### 2.2.3. Expression génique de l'HIF1$\alpha$ (Tableau 6) :

L'association d'actifs arabinogalactane / peptides de lupin a significativement inhibé l'expression du facteur de transcription HIF1$\alpha$ par des kératinocytes (54% d'inhibition).

**Tableau 6**

| | Moyenne $\Delta$CT (CT$_{HIF1-a}$ - CT$_{GAPDH}$) | Ecart type | $\Delta\Delta$CT | Expression de HIF1$\alpha$ Quantité relative | % Inhibition |
|---|---|---|---|---|---|
| Cellules contrôles | 5,5 | 1,3 | | 1 | |
| ARG 0,2% + PLU 0,02% | 6,63 | 1,22 | -1,13 | 0,456 ** | 54,3% |

** p<0,01 *vs* cellules contrôles

### 2.2.4. Expression génique de la thrombospondine-1 (Tableau 7):

L'association d'actifs arabinogalactane / peptides de lupin a significativement stimulé l'expression de la thrombospondine, facteur anti-angiogénique, par des kératinocytes (76% d'augmentation).

**Tableau 7**

| | Moyenne $\Delta$CT (CT$_{THBS-1}$ - CT$_{GAPDH}$) | Ecart type | $\Delta\Delta$CT | Expression de la THBS1 (QR) | % Stimulation |
|---|---|---|---|---|---|
| Cellules contrôles | 4,34 | 1,8 | 0 | 1 | |
| ARG 0,2% + PLU 0,02% | 3,52 | 1,44 | 0,82 | 1,765 * | 76,5% |

* p<0,05 *vs* cellules contrôles

**Conclusion**

Dans les conditions expérimentales présentées ci-dessus, nous avons pu montrer que l'association d'actifs arabinogalactane / peptides de lupin module l'expression de facteurs impliqués dans l'induction de l'angiogénèse, en faveur d'un effet anti-angiogénique. En effet, les actifs ont pu diminuer l'expression des marqueurs pro-angiogéniques (FGF2, VEGF) et augmenter l'expression d'un marqueur anti-angiogénique (THBS1). De plus, un effet inhibiteur a également été observé sur le facteur de transcription du VEGF (HIF1-$\alpha$), suggérant une régulation par les actifs en amont du VEGF *via* son facteur de transcription.

Ainsi en défavorisant l'angiogénèse, phénomène précoce de la néovascularisation, l'association arabinogalactane et peptides de lupin contribue à limiter l'activation des cellules endothéliales et la synthèse de nouveaux vaisseaux sanguins.

**2.3. Expression génique des MMP-2 et -9, marqueurs de la néo-vascularisation**

L'effet de l'association d'actifs arabinogalactane / peptides de lupin a été recherché sur des gènes impliqués dans les phénomènes moléculaires de la néo-vascularisation.

Ainsi, l'expression génique de deux Matrix Métalloproteinases a été étudiée : la MMP-2 et la MMP-9, responsables de la dégradation de la matrice extracellulaire et qui favorisent la migration des cellules endothéliales, étape de l'angiogénèse, nécessaire à la formation de nouveaux vaisseaux sanguins.

L'effet des actifs a été recherché en condition basale (pour l'étude de la MMP2) ou en préventif sur des tapis cellulaires ayant subi un stress inflammatoire induit par le PMA (pour la MMP9).

**Matériel et méthodes**

Des kératinocytes épidermiques humains normaux (NHEK) ont été pré-incubés pendant 24h en présence des actifs arabinogalactane (ARG) / peptides de lupin (PLU) aux concentrations de 0,2% / 0,02%. Le PMA a ensuite été ajouté pour un temps total de traitement de 40h. Pour l'évaluation en conditions basales, les cellules ont simplement été incubées pendant 40h en présence des actifs arabinogalactane (ARG) / peptides de lupin (PLU) aux concentrations de 0,2% / 0,02%.

A la fin du traitement, les surnageants de culture ont été éliminés et les ARN totaux ont été extraits analysés et dosés. Les cDNA néo-synthétisés relatifs aux gènes d'intérêt (MMP2, MMP9) ou aux gènes de référence ont été amplifiés sélectivement par PCR en temps réel sur iQ5 [Biorad] par la technologie SybrGreen.

Pour chaque échantillon, le niveau d'expression du gène d'intérêt a été normalisé par le niveau d'expression du gène de référence le plus stable (gène GAPDH). A partir des calculs de $\Delta Ct$ ($Ct_{\text{gène intérêt}} - Ct_{\text{gène de référence}}$) et de $\Delta\Delta Ct$ ($\Delta Ct_{\text{contrôle}} - \Delta C_{\text{traité}}$), la quantité relative du gène d'intérêt a été mesurée : $QR = (1+E)^{\Delta\Delta Ct}$, en considérant que E (l'efficacité) est égale à 1, on a donc $QR = 2^{\Delta\Delta Ct}$.

La significativité des résultats a été vérifiée sur les valeurs de $\Delta Ct$, par une analyse de variance à un facteur suivie d'un test de Dunnett (pour la MMP2) ou de Tuckey (pour la MMP9).

Le % inhibition est ainsi calculé pour MMP2 :

$$[(QR\ (contrôle) - QR\ (traitement\ actif)) / QR\ (contrôle)] *100$$

Le % inhibition est ainsi calculé pour MMP9 :

$$[(QR\ (stimulé\ PMA) - QR\ (traitement\ actif)) / QR\ (stimulé\ PMA)] *100$$

**Résultats**

**2.3.1. Expression génique de la MMP2 (Tableau 8):**

**L'association d'actifs arabinogalactane / peptides de lupin a significativement inhibé l'expression génique de la MMP2** (23% d'inhibition).

**Tableau 8**

| | Moyenne $\Delta$CT ($CT_{MMP2}$ - $CT_{GAPDH}$) | Ecart type | $\Delta\Delta$CT | **Expression de MMP2 Quantité relative** | **% Inhibition** |
|---|---|---|---|---|---|
| Cellules contrôles | 6,99 | 0,72 | | 1 | 1 |
| ARG 0,2% + PLU 0,02% | 7,38 | 0,98 | -0,39 | 0,76 * | 23,7% |
| * p<0,05 *vs* cellules contrôles | | | | | |

### 2.3.2. Expression génique de la MMP9 (Tableau 9):

Le PMA a significativement et très fortement stimulé l'expression génique de la MMP9 dans les kératinocytes. Dans ces conditions, l'association d'actifs arabinogalactane et peptides de lupin a permis d'inhiber l'expression génique de la MMP9 induite par le PMA (54% d'inhibition).

**Tableau 9**

| | Moyenne $\Delta$CT ($CT_{MMP9}$ - $CT_{GAPDH}$) | Ecart type | $\Delta\Delta$CT | Expression de MMP9 Quantité relative | % Inhibition |
|---|---|---|---|---|---|
| Cellules contrôles | 8,87 | 1,16 | | 1 | |
| Témoin stimulé (PMA) | 2,32 | 1,03 | 6,55 | 93,70 $$$ | |
| ARG 0,2% + PLU 0,02% | 3,46 | 0,7 | 5,41 | 42,51 * | 54,6% |
| $$$ p<0,001 vs cellules contrôles<br>* p<0,05 *vs* témoin stimulé | | | | | |

### Conclusion

Dans les conditions expérimentales décrites ci-dessus, un effet inhibiteur de l'expression des protéinases matricielles MMP2 et MMP9 a été montré pour l'association arabinogalactane / peptides de lupin.

Ainsi, en réduisant la dégradation de la matrice extracellulaire, les actifs contribuent à limiter l'afflux des cellules endothéliales, étape précoce de la néo-vascularisation.

### 3. Activité sur un paramètre inflammatoire : IL8

### Matériel et méthodes

Des kératinocytes humains (de lignée NCTC 2544) ont été pré-incubés pendant 24 heures avec les actifs arabinogalactane (ARG) / peptides de lupin (PLU) aux concentrations de 0,2% / 0,02% ; ou avec la molécule de référence dexaméthasone.

L'inflammation a ensuite été induite par traitement au PMA pendant 24 heures, toujours en présence des actifs ou de la molécule de référence.

A la fin de l'incubation, les quantités d'interleukine-8 (IL8) présentes dans les surnageants de culture ont été mesurées par ELISA.

La significativité des résultats a été vérifiée par une analyse de variance à un facteur suivie d'un test de Tuckey.

Le % inhibition est calculé selon la formule suivante :

$$[\text{(stimulé PMA)} - \text{(traitement actif)} / \text{(stimulé PMA)}] * 100$$

**Résultats (Tableau 10)**

Le traitement par le PMA a nettement stimulé la libération d'IL8 par les kératinocytes, la dexaméthasone a nettement inhibé cette libération (-81%), ces résultats valident l'essai. L'association d'actifs arabinogalactane + peptides de lupin a significativement inhibé le relargage d'IL8 induit par le PMA (33% d'inhibition).

**Tableau 10**

| IL8 (ng/ml) | | |
|---|---|---|
| Cellules contrôles | 0,1033 +/- 0,002 | |
| Témoin stimulé (PMA) | 30,60 +/- 1,888 | $$$ |
| Dexaméthasone $10^{-7}$M | 5,967 +/- 0,203 | 80,5% *** |
| ARG 0,2% + PLU 0,02% | 20,40 +/- 1,559 | 33,3% ** |

$$$ p<0,001 *vs* cellules contrôles
*** p<0,001 - ** p<0,01 *vs* témoin stimulé

**Conclusion**

Dans ces conditions expérimentales, l'association d'actifs arabinogalactane + peptides de lupin a présenté un effet modulateur du médiateur inflammatoire, l'IL8.

**Conclusion générale**

L'arabinogalactane présente les effets suivants:

- une action inhibitrice sur l'expression de LL37 (tableau 1) et sur l'expression du VEGF (tableau 4),
- une limitation de la prolifération des cellules endothéliales suite à stimulation par le VEGF (tableau 3)

Ceci en fait un bon principe actif pour la prévention et/ou le traitement des rougeurs.
L'association de cet actif avec des peptides de lupin permet d'obtenir des résultats bien meilleurs encore, par une action sur la plupart des facteurs impliqués dans les rougeurs cutanées tels que les facteurs immunitaires et inflammatoires et les facteurs vasculaires et dermiques.

**4. Exemples de compositions pour application par voie topique**

Les inventeurs présentent ci-dessous plusieurs compositions pour application par voie topique. L'arabinogalactane et les peptides de lupin peuvent être incorporés à divers produits cosmétiques tels que des eaux nettoyantes, des émulsions, des crèmes, des laits, des lotions, des produits moussants et sprays, dont les compositions sont présentées ci-dessous.

**EMULSION ANTI-ROUGEURS SPF 20**

| | |
|---|---|
| AQUA (WATER) | QSP 100% |
| GLYCERIN | De 1 à 15 % |
| DICAPRYLYL CARBONATE | De 1 à 10 % |
| DICAPRYLYL ETHER | De 1 à 10 % |
| CAPRYLIC/CAPRIC TRIGLYCERIDE | De 1 à 10% |
| GLYCERYLSTEARATE | De 1 à 5% |
| FILTRES SOLAIRES CHIMIQUES | De 1 à 10% |
| BUTYROSPERMUM PARKII (SHEA) BUTTER | De 1 à 5% |

(suite)

| CETYL ALCOHOL | De 1 à 5% |
|---|---|
| DEXTRIN PALMITATE | De 0 à 2% |
| LAURETH-23 | De 0 à 2% |
| HYDROXYETHYL ACRYLATE/SODIUM ACRYLOYLDIMETHYL TAURATE COPOLYMER | De 0 à 2% |
| PEG-75 STEARATE | De 0 à 2% |
| CERA ALBA (BEESWAX) | De 0 à 2% |
| CONSERVATEURS | De 0 à 2% |
| CETETH-20 | De 0 à 0.5% |
| STEARETH-20 | De 0 à 0.5% |
| DECYL GLUCOSIDE | De 0 à 0.5% |
| XANTHAN GUM | De 0 à 0.5% |
| TOCOPHERYL ACETATE | De 0 à 0.5% |
| CETEARYL ALCOHOL | De 0 à 0.5% |
| CETYL PALMITATE | De 0 à 0.5% |
| COCOGLYCERIDES | De 0 à 0.5% |
| EXTRAIT PEPTIDIQUE DE QUINOA | De 0,01 à 10 % |
| CYCLOCERAMIDES | De 0,01 à 10 % |
| **ARABINOGALACTANE** | De 0,01 à 10% |
| CONCENTRAT D'HUILE DE TOURNESOL | De 0,01 à 10 % |
| COLORANT | De 0 à 0.5% |
| CITRIC ACID | De 0 à 0.5% |

**EMULSION HYDRATANTE ANTIROUGEURS**

| AQUA (WATER) | QSP 100% |
|---|---|
| GLYCERIN | De 1 à 15 % |
| PROPANEDIOL DICAPRYLATE | De 1 à 15 % |
| DICAPRYLYL CARBONATE | De 1 à 15 % |
| GLYCERYLSTEARATE CITRATE | De 1 à 5% |
| CONSERVATEURS | De 1 à 5% |
| CETYL ALCOHOL | De 1 à 5% |
| ACRYLATES/C10-30 ALKYL ACRYLATE CROSSPOLYMER | De 0 à 2% |
| GLYCERYL CAPRYLATE | De 0 à 2% |
| SODIUM STEAROYL GLUTAMATE | De 0 à 2% |
| TOCOPHERYL ACETATE | De 0 à 2% |
| SODIUM HYDROXIDE | De 0 à 2% |
| EXTRAIT PEPTIDIQUE DE QUINOA | De 0,01 à 10 % |
| CYCLOCERAMIDES | De 0,01 à 10 % |

(suite)

| | |
|---|---|
| PEPTIDES DE LUPIN | De 0,01 à 10 % |
| **ARABINOGALACTANE** | De 0,01 à 10 % |
| CONCENTRAT D'HUILE DE TOURNESOL | De 0,01 à 10% |
| BISABOLOL | De 0 à 2% |

**EAU NETTOYANTE PEAU SENSIBLE**

| Nom commercial ou INCI | % |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |
| CYCLOCERAMIDES | De 0,01 à 10 % |
| **ARABINOGALACTANE** | De 0,01 à 10 % |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1% |
| POLOXAMER 184 | De 1 à 5 % |

**EMULSION ANTI-AGE**

| Nom commercial ou INCI | % |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |

(suite)

| Nom commercial ou INCI | % |
|---|---|
| **ARABINOGALACTANE** | De 0,01 à 10 % |
| PEPTIDES DE LUPIN | De 0,01 à 10 % |
| PEPTIDES DE MACA | De 0,01 à 10 % |
| ISONONYL ISONONANOAT | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

**EMULSION RESTRUCTURANTE**

| Matière première / Nom commercial ou INCI | % |
|---|---|
| HYDROGENATED POLYDECENE | De 5 à 20% |
| LAURYLGLUCOSIDE-GLYSTEARATE | De 1 à 5% |
| DICAPRYLYL CARBONATE | De 1 à 5 % |
| GLYCEROL | De 5 à 20% |
| CARBOPOL | De 0 à 1% |
| GOMME XANTHANE | De 0 à 1 % |
| ACIDE ASIATIQUE | De 0 à 1 % |
| VITAMINE B5 | De 0 à 5 % |
| **ARABINOGALACTANE** | De 0,01 à 10 % |
| EXTRAIT PEPTIDIQUE DE QUINOA | De 0,01 à 10% |
| LESSIVE SOUDE | De 0 à 1 % |
| CONSERVATEURS | De 0 à 1 % |
| ACIDE CITRIQUE | De 0 à 1 % |
| EAU PURIFIÉE | QSP 100 % |

**LAIT pour PEAU SECHE, ATOPIQUE**

| Matière première / Nom commercial ou INCI | % |
|---|---|
| HUILE AMANDE DOUCE | De 1 à 5 % |

(suite)

| Matière première / Nom commercial ou INCI | % |
|---|---|
| HUILE MAIS | De 1 à 5 % |
| ACIDE STEARIQUE | De 1 à 5 % |
| ALCOOL CETYLIQUE C16 C18 | De 0 à 1 % |
| ANTIMOUSSE 70414 | De 0 à 1 % |
| ALCOOL LAURIQUE 11OE | De 1 à 5 % |
| MONOLAURATE PEG 300 | De 0 à 1 % |
| MONOLEATE DE GLYCEROL | De 0 à 1 % |
| MONOSTEARATE DE GLYCEROL | De 1 à 5 % |
| VITAMINE B12 | De 0 à 5 % |
| **ARABINOGALACTANE** | De 0,1 à 10 % |
| CONCENTRAT D'HUILE DE TOURNESOL | De 0,01 à 10 % |
| PEPTIDES DE LUPIN | De 0,01 à 10 % |
| CONSERVATEURS | De 0 à 1 % |
| ACIDE CITRIQUE | De 0 à 1 % |
| CITRATE TRISODIQUE | De 0 à 1 % |
| EAU PURIFIEE | QSP 100 % |
| PARFUM | De 0 à 1 % |
| HUILE ARACHIDE | De 1 à 5 % |
| HUILE PALMISTE HYDROGENEE | De 1 à 5 % |

**SPRAY SOLAIRE SPF 50+**

| Matière première / Nom commercial ou INCI | % |
|---|---|
| CAPRYLO CAPRATE DE GLYCEROL | De 5 à 20% |
| CYCLOPENTASILOXANE | De 10 à 20% |
| DICAPRYLYL CARBONATE | De 5 à 20% |
| TINOSORB S | De 1 à 10% |
| OXYDE DE TITANE 100 | De 10 à 20% |
| HECTORITE | De 0 à 5% |
| ALPHA TOCOPHEROL | De 0 à 2% |
| LAURYLGLUCOSIDE-GLYSTEARATE | De 0 à 10% |
| EAU PURIFIÉE B4 | QSP 100% |
| ACIDE CITRIQUE | De 0 à 2% |
| PENTYLENE GLYCOL | De 0 à 5% |
| GLYCEROL | De 0 à 5% |
| GOMME XANTHANE | De 0 à 2% |
| **ARABINOGALACTANE** | **De 0,01 à 10 %** |

(suite)

| Matière première / Nom commercial ou INCI | % |
|---|---|
| ALOE VERA | De 0 à 1% |
| GLUCONATE ZINC | De 0 à 1% |
| CONSERVATEURS | De 0 à 2% |
| TINOSORB M | De 1 à 10% |

**5. Exemples de formulations de compositions pour administration par voie orale**

L'arabinogalactane et les peptides de Lupin sont intégrés à des compositions orales, dans des compositions permettant l'administration de 50 mg à 200 mg par jour.

**1/ Composition anti-rougeurs sous forme de capsules molles**

| | |
|---|---|
| - ARABINOGALACTANE | 30 mg |
| - PEPTIDES DE LUPIN | 30 mg |
| - Huile d'Awara | 60 mg |
| - Huile de colza riche en insaponifiable | 300 mg |
| - Vitamine du groupe B (B1, B2, B3, B5, B6, B9, B12), | QSP 100% des AJR |
| - Tocotriénols | QSP 50 % AJR |
| - Vitamine E | |
| - Cire d'abeille | |
| - Lécithine de soja | |
| - Gélatine alimentaire | |
| - Glycérine QSP 1 capsule molle | |

Cette composition est administrée de 4 à 6 capsules de 500 mg par jour.

**2/ Exemples en stick poudre apaisante**

| | |
|---|---|
| - **ARABINOGALACTANE** | 100 mg |
| - PEPTIDES DE LUPIN | 100 mg |
| - Extrait de thé riche en polyphénols | 100 mg |
| - Extrait de raisin riche en OPC | 50 mg |
| - Bétaglucanes d'origine végétale | 100 mg |
| - Gomme xanthane | 1 mg |
| - ascorbate de sodium | 0,3 mg |
| - maltodextrine | QSP 5 g. |

Cette composition est administrée 2 fois par jour.

**Références**

[1] Yamasaki K and Gallo RL. The molecular pathology of rosacea. 2009, J Dermatol Science; 55:77-81.
[2] Koczulla R, von Degenfeld G, Kupatt C, Krotz F, Zahler S, Gloe T et al. An angiogenic role for the human peptide antibiotic LL37/hCAP-18. 2003, J Clin Invest; 111:1665-72.

[3] Rodriguez-Martinez S, Cancino-Diaz JL, Vargas-Zuniga LM, Cancino-Diaz ME. LL-37 regulates the overexpression of vascular endothelial growth factor (VEGF) and c-IAP-2 in human keratinocytes. 2008, Inter J Dermatol; 47:457-62.

[4] Yamasaki K, Schauber J, Coda A, Lin H, Dorschner RA, Schechter NM et al. Kallikrein-mediated proteolysis regulates the antimicrobial effects of cathelicidins in skin. 2006, FASEB J; 20:2068-80.

[5] Yamasaki K, Di Nardo A, Bardan A, Murakami M, Ohtake T, Coda A et al. Increased serine protease activity and cathelicidin promotes skin inflammation in rosacea. 2007, Nat Med; 13:975-80.

[6] Yamasaki K, Kanada K, Macleod DT, Borkowski AW, Morizane S, Nakatsuji et al. TLR2 expression is increased in Rosacea and stimulates enhanced serine protease production by keratinocytes. 2011, J Invest Dermatol; 131:688-697

[7] Bevins CL and Liu FT. Rosacea : skin innate immunity gone away, 2007, Nat Med ; 13 :904-906

[8] Guzman-Sanchez Da, Ishiuji Y, Patel T, Fountain J, Chan YH, Yosipovitch G. enhanced skin blood flow and sensitivity to noxious heat stimuli in papulo-pustular rosacea. 2007, J Am Acad dermatol;

[9] Laquer V, Hoang V, Nguyen A, Kelly KM. Angiogenesis in cutaneous disease: Part II. 2009, J Am Acad Dermatol; 61: 945-58.

[10] Stetler-Stevenson WG. Matrix metalloproteinases in angiogenesis: a moving target for therapeutic interventions. 1999, J Clin Invest; 103:1237-41

[11] Gomaa AH, Yaar M, Eyada MM, Bhawan J. Lymphangiogenesis and angiogenesis in non-phymatous rosacea. 2007, J Cutan Pathol; 34:748-53.

[12] Smith JR, Lanier VB, Braziel RM, Falkenhagen KM, White C, Rosenbaum JT. Expression of vascular endothelial growth factor and its receptors in rosacea. 2007, Br J Ophthalmol; 91:226-229.

[13] Tokumaru S, Sayama K, Shirakata Y, Komatsuzawa H, Ouhara K, Hanakawa Y et al. Induction of keratinocyte migration via transactivation of the epidermal growth factor receptor by the antimicrobial peptide LL-37. 2005, J Immunol; 175:4662-8.

## Revendications

1. Composition comprenant de l'arabinogalactane pour utilisation dans la prévention et/ou le traitement des rougeurs de la peau chez une personne susceptible de former ou ayant des rougeurs.

2. Composition pour utilisation selon la revendication 1, **caractérisée en ce que** ladite composition est administrée par voie topique.

3. Composition pour utilisation selon la revendication 1, **caractérisée en ce que** ladite composition est administrée par voie orale.

4. Composition pour utilisation selon l'une des revendications 1 à 3, **caractérisée en ce que** l'arabinogalactane est extrait de mélèze.

5. Composition pour utilisation selon l'une des revendications 1 à 4, **caractérisée en ce que** l'arabinogalactane est présent dans ladite composition dans une proportion comprise entre 0,01% et 10% en poids par rapport au poids total de la composition.

6. Composition pour utilisation selon l'une des revendications 1 à 5, **caractérisée en ce que** ladite composition comprend au moins un autre agent anti-rougeurs choisi parmi : les peptides de lupin, le perméthol, la génistéine, l'esculoside, le sulfate de dextran, l'hespéridine méthylchalcone, les rétinoïdes, la licochalcone, l'oxyméthazoline, la kinétine, l'extrait de réglisse, la vitamine P-like, l'extrait de petit houx, le *Sophora japonica,* l'extrait d'Hamamélis, le ruscus, l'acide fucidique, la pénicilline, les tétracyclines, la pristinamycine, l'érythromycine, la clindamycine, la mupirocine, la minocycline, la doxycycline, les polyphénols dont les tanins, les acides phénoliques, les anthocyanes, les procyanidols, les flavonoïdes, l'extrait de thé vert, l'extrait de fruits rouges, l'extrait de cacao, l'extrait de raisin, l'extrait de *Passiflora incarnata,* l'extrait de *Citrus.*

7. Composition pour utilisation selon la revendication 6, **caractérisée en ce que** ladite composition comprend de l'arabinogalactane et des peptides de lupin.

8. Composition pour utilisation selon la revendication 7, **caractérisée en ce que** ladite composition comprend entre 0,01% et 10%, préférentiellement 2% d'arabinogalactane

9. Composition pour utilisation selon la revendication 6, **caractérisée en ce que** ladite composition comprend entre 0,01 % et 10%, préférentiellement 0,2 % de peptides de lupin.

10. Arabinogalactane pour utilisation pour prévenir et/ou traiter l'apparition des rougeurs sur la peau.

11. Combinaison d'arabinogalactane et de peptides de lupin pour prévenir et/ou traiter l'apparition des rougeurs sur la peau.

12. Composition comprenant de l'arabinogalactane pour son utilisation dans la prévention et/ou le traitement de la rosacée.

13. Composition comprenant de l'arabinogalactane et des peptides de lupin pour son utilisation dans la prévention et/ou le traitement de la rosacée.

**Patentansprüche**

1. Zusammensetzung, umfassend Arabinogalaktan zur Verwendung bei der Prävention und / oder der Behandlung von Rötungen der Haut bei einer Person, die Rötungen bilden oder haben kann.

2. Zusammensetzung zur Verwendung gemäß Anspruch 1, **dadurch gekennzeichnet, dass** die genannte Zusammensetzung auf topischem Weg verabreicht wird.

3. Zusammensetzung zur Verwendung gemäß Anspruch 1, **dadurch gekennzeichnet, dass** die genannte Zusammensetzung auf oralem Weg verabreicht wird.

4. Zusammensetzung zur Verwendung gemäß Anspruch 1 bis 3, **dadurch gekennzeichnet, dass** das Arabinogalaktan aus Lärche extrahiert ist.

5. Zusammensetzung zur Verwendung gemäß Anspruch 1 bis 4, **dadurch gekennzeichnet, dass** das Arabinogalaktan in der genannten Zusammensetzung in einem zwischen 0,01 Gew.-% und 10 Gew.-% inbegriffenen Anteil im Verhältnis zum Gesamtgewicht der Zusammensetzung vorhanden ist.

6. Zusammensetzung gemäß Anspruch 1 bis 5, **dadurch gekennzeichnet, dass** die genannte Zusammensetzung wenigstens einen anderen Wirkstoff gegen Rötungen umfasst, der ausgewählt ist aus: Lupinenpeptiden, Permethol, Genistein, Esculosid, Dextransulfat, Hesperidinmethylchalcon, Retinoiden, Licochalcon, Oxymethazolin, Kinetin, Lakritzextrakt, Vitamin P-like, Extrakt aus Mäusedorn, *Sophora japonica,* Extrakt aus Hamamelis, Ruscus, Fusidensäure, Penicilin, Tetracyclin, Pristinamycin, Erythromycin, Clindamycin, Mupirocin, Minocyclin, Doxycyclin, Polyphenole, darunter Tannine, Phenolsäuren, Anthocyane, Procyanidole, Flavonoide, Extrakt aus grünem Tee, Extrakt aus roten Früchten, Kakaoextrakt, Traubenextrakt, Extrakt aus *Passiflora incarnata,* Extrakt aus *Citrus.*

7. Zusammensetzung zur Verwendung gemäß Anspruch 6, **dadurch gekennzeichnet, dass** die genannte Zusammensetzung Arabinogalaktan und Lupinenpeptide umfasst.

8. Zusammensetzung zur Verwendung gemäß Anspruch 7, **dadurch gekennzeichnet, dass** die genannte Zusammensetzung zwischen 0,01 % und 10 %, bevorzugt 2 % Arabinogalaktan umfasst.

9. Zusammensetzung zur Verwendung gemäß Anspruch 6, **dadurch gekennzeichnet, dass** die genannte Zusammensetzung zwischen 0,01 % und 10 %, bevorzugt 0,2 % Lupinenpeptide umfasst.

10. Arabinogalaktan zur Verwendung zwecks Prävention und / oder Behandlung des Auftretens von Rötungen der Haut.

11. Verbindung von Arabinogalaktan und Lupinenpeptiden zwecks Prävention und / oder Behandlung des Auftretens von Rötungen der Haut.

12. Verbindung, umfassend Arabinogalaktan zur Verwendung bei der Prävention und / oder Behandlung von Rosazea.

13. Verbindung, umfassend Arabinogalaktan und Lupinenpeptide für ihre Verwendung bei der Prävention und / der Behandlung von Rosazea.

**Claims**

1. Composition comprising arabinogalactan for use in the prevention and/or treatment of redness of the skin in individuals likely to form or experience redness.

2. Composition for use according to claim, 1 **characterised in that** said composition is administered topically.

3. Composition for use according to claim 1, **characterised in that** said composition is administered orally.

4. Composition for use according to any of claims 1 to 3, **characterised in that** the arabinogalactan is extracted from larch.

5. Composition for use according to any of claims 1 to 4, **characterised in that** the arabinogalactan is present in said composition in a proportion of between 0.01% and 10% by weight in relation to the total weight of the composition.

6. Composition for use according to any of claims 1 to 5, **characterised in that** said composition comprises at least one other anti-redness agent selected from the group consisting of: lupin peptides, permethol, genistein, esculoside, dextran sulphate, hesperidin methyl chalcone, retinoids, licochalcone, oxymetazoline, kinetin, licorice extract, vitamin P-like substances, butcher's broom extract, *Sophora japonica, Hamamelis* extract, *Ruscus,* fucidic acid, penicillin, tetracyclines, pristinamycin, erythromycin, clindamycin, mupirocin, minocycline, doxycycline, polyphenols including tannins, phenolic acids, anthocyanins, procyanidols, flavonoids, green tea extract, red berries extract, cocoa extract, grape extract, *Passiflora incarnata* extract, and *Citrus* extract.

7. Composition for use according to claim 6, **characterised in that** said composition comprises arabinogalactan and lupin peptides.

8. Composition for use according to claim 7, **characterised in that** said composition comprises between 0.01% and 10%, preferentially 2% of arabinogalactan.

9. Composition for use according to claim 6, **characterised in that** said composition comprises between 0.01% and 10%, preferentially 0.2% of lupin peptides.

10. Arabinogalactan for use in preventing and/or treating outbreaks of redness of the skin.

11. Combination of arabinogalactan and of lupin peptides to prevent and/or treat outbreaks of redness of the skin.

12. Composition comprising arabinogalactan for use in prevention and/or treatment of rosacea.

13. Composition containing arabinogalactan and lupin peptides for use in the prevention and/or treatment of rosacea.

**Peau normale**

**Peau sujette aux rougeurs**

**Agression chimique
physique**  **Microbes**  **U**

**↗↗ TLR**

**Précurseur**

**Précurseur**

**Processing
normal**

**Kallikréine ↗↗**

**Production normale**

**Production variants LL37**

**Antimicrobien**

**cicatrisation**

**Inflammation↗↗**

**Antimicrobien**

**Angiogénèse ↗↗**

**Immunité innée efficace**

**Troubles vasculaires +**

Figure 1

**Expression du gène LL37**

**Expression du gène VEGF**

Figure 2

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009150257 A
- WO 2010007175 A
- FR 2885301
- FR 2855050
- WO 0062789 A
- EP 1600461 A
- WO 2007099997 A
- EP 0866808 A
- FR 2836378
- WO 2010020379 A
- WO 2010052327 A
- WO 2005102259 A
- WO 2008080974 A
- WO 0121150 A
- WO 2004050052 A
- WO 2004050079 A
- WO 2004112741 A
- WO 2004012496 A
- WO 2004012752 A
- WO 2004016106 A
- WO 2007057439 A

- WO 9847479 A
- WO 0121605 A
- WO 0151596 A
- WO 2005105123 A
- WO 2004112742 A
- WO 2008009709 A
- WO 2005115421 A
- WO 0152837 A
- WO 0206205 A
- FR 1061055
- FR 1061047
- FR 1061051
- FR 2822821
- FR 2857596
- FR 0958525
- FR 0958529
- FR 0955344
- WO 2011012612 A
- FR 0955343
- WO 2011012615 A

**Littérature non-brevet citée dans la description**

- **YAMASAKI K ; GALLO RL.** The molecular pathology of rosacea. *J Dermatol Science,* 2009, vol. 55, 77-81
- **KOCZULLA R ; VON DEGENFELD G ; KUPATT C ; KROTZ F ; ZAHLER S ; GLOE T et al.** An angiogenic role for the human peptide antibiotic LL37/hCAP-18. *J Clin Invest,* 2003, vol. 111, 1665-72
- **RODRIGUEZ-MARTINEZ S ; CANCINO-DIAZ JL ; VARGAS-ZUNIGA LM ; CANCINO-DIAZ ME.** LL-37 regulates the overexpression of vascular endothelial growth factor (VEGF) and c-IAP-2 in human keratinocytes. *Inter J Dermatol,* 2008, vol. 47, 457-62
- **YAMASAKI K ; SCHAUBER J ; CODA A ; LIN H ; DORSCHNER RA ; SCHECHTER NM et al.** Kallikrein-mediated proteolysis regulates the antimicrobial effects of cathelicidins in skin. *FASEB J,* 2006, vol. 20, 2068-80
- **YAMASAKI K ; DI NARDO A ; BARDAN A ; MURAKAMI M ; OHTAKE T ; CODA A et al.** Increased serine protease activity and cathelicidin promotes skin inflammation in rosacea. *Nat Med,* 2007, vol. 13, 975-80

- **YAMASAKI K ; KANADA K ; MACLEOD DT ; BORKOWSKI AW ; MORIZANE S ; NAKATSUJI et al.** TLR2 expression is increased in Rosacea and stimulates enhanced serine protease production by keratinocytes. *J Invest Dermatol,* 2011, vol. 131, 688-697
- **BEVINS CL ; LIU FT.** Rosacea : skin innate immunity gone away. *Nat Med,* 2007, vol. 13, 904-906
- **GUZMAN-SANCHEZ DA ; ISHIUJI Y ; PATEL T ; FOUNTAIN J ; CHAN YH ; YOSIPOVITCH G.** enhanced skin blood flow and sensitivity to noxious heat stimuli in papulo-pustular rosacea. *J Am Acad dermatol,* 2007
- **LAQUER V ; HOANG V ; NGUYEN A ; KELLY KM.** Angiogenesis in cutaneous disease. *J Am Acad Dermatol,* 2009, vol. 61, 945-58
- **STETLER-STEVENSON WG.** Matrix metalloproteinases in angiogenesis: a moving target for therapeutic interventions. *J Clin Invest,* 1999, vol. 103, 1237-41
- **GOMAA AH ; YAAR M ; EYADA MM ; BHAWAN J.** Lymphangiogenesis and angiogenesis in non-phymatous rosacea. *J Cutan Pathol,* 2007, vol. 34, 748-53

- **SMITH JR ; LANIER VB ; BRAZIEL RM ; FALKEN-HAGEN KM ; WHITE C ; ROSENBAUM JT.** Expression of vascular endothelial growth factor and its receptors in rosacea. *Br J Ophthalmol,* 2007, vol. 91, 226-229

- **TOKUMARU S ; SAYAMA K ; SHIRAKATA Y ; KO-MATSUZAWA H ; OUHARA K ; HANAKAWA Y et al.** Induction of keratinocyte migration via transactivation of the epidermal growth factor receptor by the antimicrobial peptide LL-37. *J Immunol,* 2005, vol. 175, 4662-8